(19) Europäisches Patentamt
European Patent Office
Office européen des brevets

(11) Numéro de publication: **0 301 946**
**A1**

(12) # DEMANDE DE BREVET EUROPEEN

(21) Numéro de dépôt: **88401892.0**

(51) Int. Cl.4: **C 07 D 249/12**

(22) Date de dépôt: **21.07.88**

(30) Priorité: **31.07.87 FR 8710869**

(43) Date de publication de la demande:
**01.02.89 Bulletin 89/05**

(84) Etats contractants désignés:
**AT BE CH DE ES FR GB IT LI NL**

(71) Demandeur: **SOCIETE NATIONALE DES POUDRES ET EXPLOSIFS**
**12, quai Henri IV**
**F-75181 Paris Cédex 04 (FR)**

(72) Inventeur: **Senet, Jean-Pierre**
**79, rue de la Gare Herbeauvilliers-Buthiers**
**F-77760 La Chapelle la Reine (FR)**

**Wolf, Patrick c/o Dijkstra**
**rue du Tertre Saint André Gazonfier**
**F-72000 Le Mans (FR)**

**Wooden, Gary Paul**
**Bâtiment 7C Résidence de Bel Air**
**F-91540 Mennecy (FR)**

(74) Mandataire: **Pech, Bernard et al**
**Sté nationale des poudres et explosifs 12, quai Henri IV**
**F-75181 Paris Cédex 04 (FR)**

(54) **Nouveau procédé de préparation des 1,2,4-triazol-3-ones.**

(57) L'invention concerne ou nouveau procédé de préparation des 1,2,4-triazol-3-ones de formule :

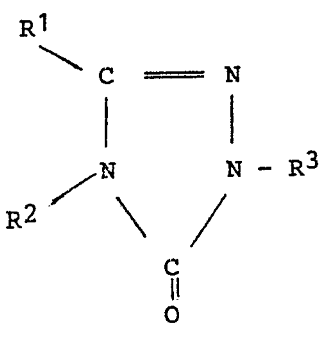

dans laquelle $R^1$ représente un radical aliphatique, cycloaliphatique, phényle, naphtyle ou hétérocyclique, $R^2$ représente un atome d'hydrogène, un radical aliphatique, cycloaliphatique, phényle, naphtyle ou hétérocyclique, et $R^3$ représente un atome d'hydrogène, un radical alkyle, phényle ou naphtyle substitué qui consiste à faire réagir une monoacylhydrazine avec du phosgène pour obtenir une 1,3,4-oxadiazolin-2-one sur laquelle on fait réagir un composé de formule $R^2NH_2$ pour obtenir un acylsemicarbazide et à cycliser ce dernier par traitement alcalin.

Les 1,2,4-triazol-3-ones sont très utiles comme intermédiaires dans la fabrication de médicaments, d'herbicides et de polymères.

EP 0 301 946 A1

**Description**

## Nouveau procédé de préparation des 1,2,4-triazol-3-ones

L'invention concerne un nouveau procédé de préparation des 1,2,4-triazol-3-ones.

Les 1,2,4-triazol-3-ones sont des intermédiaires très utiles, en particulier pour la fabrication de composés à usage phytosanitaire ou pharmaceutique.

Quelques procédés ont été proposés pour les préparer. Les plus anciens utilisent comme composés de départ les isocyanates.

Ainsi dans Annalen der Chemie 675 (1964) page 180 à 188 il est décrit un procédé de préparation des composés de formule

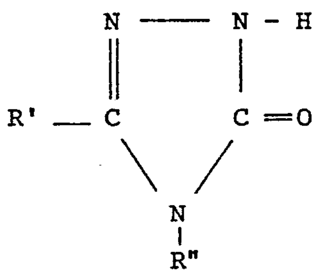

dont les différentes étapes sont les suivantes :
- réaction d'un hydrazide avec un isocyanate pour obtenir un acylsemicarbazide,
- puis cyclisation de l'acylsemicarbazide obtenu par chauffage en milieu alcalin, selon le schéma réactionnel

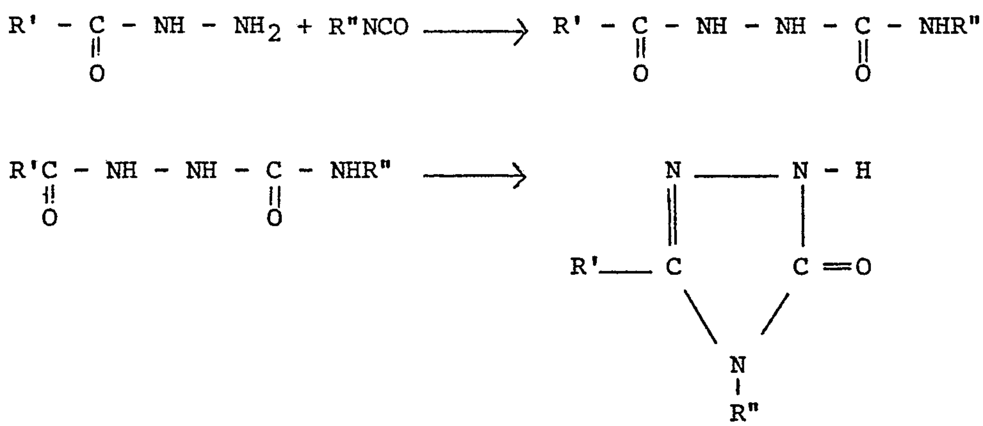

La mise en oeuvre de ce procédé est délicate parce que les isocyanates d'alkyles inférieurs ont une toxicité très élevée, en particulier ceux de méthyle et d'éthyle, et leur utilisation a même été abandonnée à la suite d'accidents récents. Ou bien ces isocyanates sont eux-mêmes difficiles à préparer comme dans le cas de l'isocyanate de phénoxyéthyle

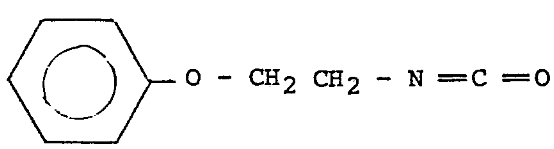

Selon un autre procédé (brevet FR N° 2 555 582) une triazolone, telle que la 5-éthyle-4-(2-phénoxyéthyl)-1,2,4-triazolone est obtenue au moyen de cinq étapes qui sont :

a)

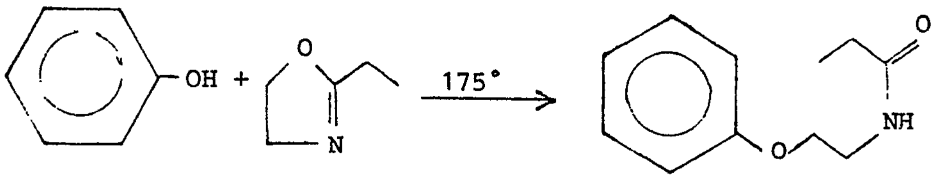


b)

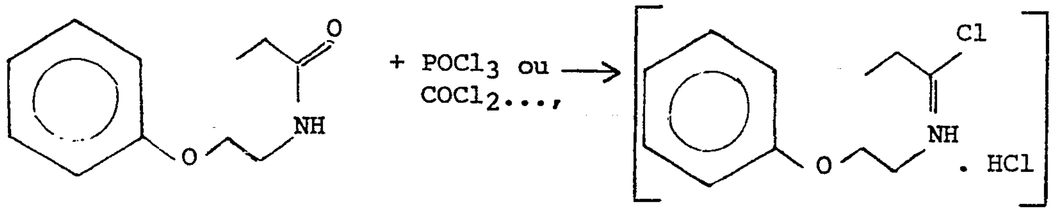


c)

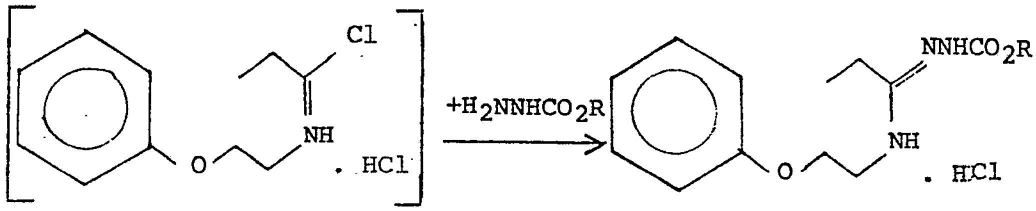


d)

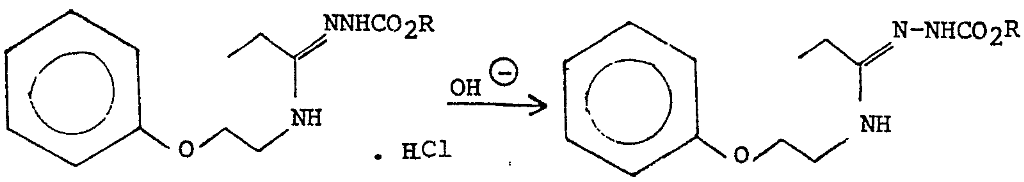


e)

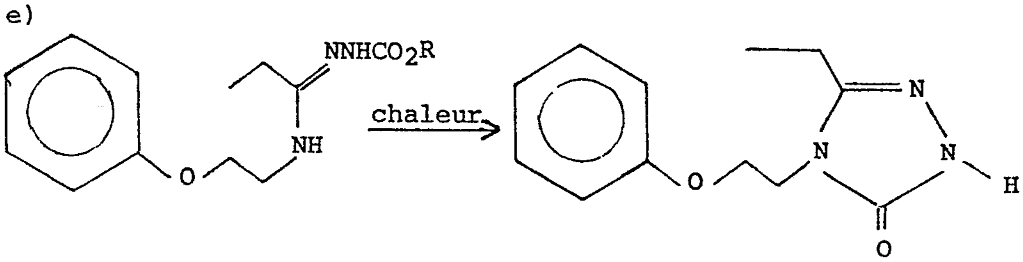

Ce procédé est long, il implique l'isolement de trois intermédiaires et par conséquent demande beaucoup de main d'oeuvre. De plus, du fait de la multiplicité des étapes, le rendement d'ensemble ne dépasse pas 40 à 55 %.

Le produit obtenu doit être purifié par alcalinisation à 100°C, filtration sur celite et charbon actif, acidification, filtration et séchage, ce qui entraîne de nouvelles dépenses de main d'oeuvre et une perte de temps supplémentaire.

Il existe par conséquent un besoin d'un procédé général de fabrication de triazolone avec le moins d'étapes et de traitements possibles et qui utilise comme composés de départ des produits simples et non toxiques.

Le procédé selon l'invention répond à ce besoin.

Selon le procédé de l'invention on prépare des 1,2,4-triazol-3-ones de formule générale :

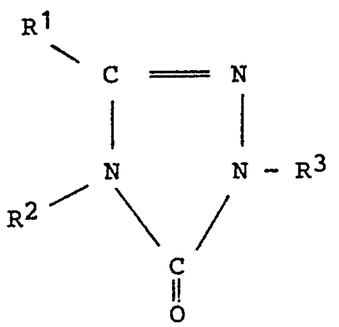

dans laquelle

$R^1$ représente

- un atome d'hydrogène
- un radical aliphatique en $C_1$ à $C_{20}$, linéaire ou ramifié, substitué ou non par un ou plusieurs substituants choisis parmi les atomes de fluor ou de chlore et le radical phényle,
- un radical cycloaliphatique en $C_3$ à $C_7$, substitué ou non par un ou plusieurs substituants choisis parmi les atomes de fluor ou de chlore, les radicaux aliphatiques en $C_1$ à $C_4$ et phényle,

$R^2$ représente

- un atome d'hydrogène,
- un radical aliphatique en $C_1$ à $C_{20}$, linéaire ou ramifié,substitué ou non par un ou plusieurs substituants choisis parmi les radicaux alkoxy en $C_1$ à $C_4$, phényle et phénoxy,
- un radical cycloaliphatique en $C_3$ à $C_7$, substitué ou non par un ou plusieurs substituants choisis parmi les radicaux aliphatiques en $C_1$ à $C_4$ et alkoxy en $C_1$ à $C_4$, phényle et phénoxy,
- un radical phényle ou naphtyle substitué ou non par un groupe nitro ou phénoxy et/ou par un à trois substituants choisis parmi les atomes de fluor, chlore ou brome, les radicaux aliphatiques en $C_1$ à $C_4$ et alkoxy en $C_1$ à $C_4$,
- un radical hétérocyclique à 5 ou 6 chaînons, saturé ou insaturé, les hétéroatomes étant choisis parmi l'oxygène, l'azote ou le soufre,

$R^3$ représente

- un atome d'hydrogène,
- un radical alkyle en $C_1$ à $C_6$, linéaire ou ramifié,
- un radical phényle ou naphtyle, substitué ou non par un à trois atomes de fluor, chlore ou brome, et/ou un à trois radicaux alkyle en $C_1$ à $C_4$, et/ou un à deux radicaux alkoxy en $C_1$ à $C_4$, linéaire ou ramifié, et/ou un radical $-CF_3$, et/ou un radical $-COOCH_3$ ou $-COOC_2H_5$, et/ou un à deux groupes $-NO_2$, et/ou un groupe de formule $R^4SO_2NH-$, dans laquelle $R^4$ représente un radical alkyle en $C_1$ à $C_4$, et/ou un groupe de formule $NR^5R^6CONH-$dans laquelle $R^5$ et $R^6$ représentent des radicaux méthyle ou éthyle,

au moyen des étapes suivantes :

a) réaction d'une monoacylhydrazine de formule

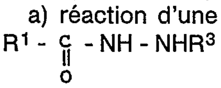 (II)

dans laquelle $R^1$ et $R^3$ ont les significations précédentes, avec du phosgène, dans un solvant ou un mélange de solvants inertes vis à vis du phosgène et à une température comprise entre 0° et 130°C, pour obtenir une 1,3,4-oxadiazolin-2-one de formule

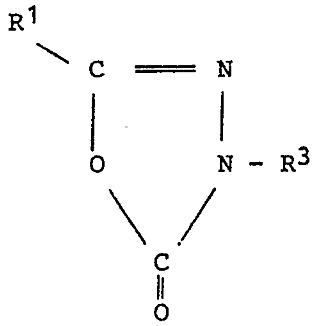

(III)

b) réaction du composé (III) précédemment obtenu avec un composé de formule $R^2NH_2$ (IV) dans laquelle $R^2$ a les significations précédentes, éventuellement dans un solvant ou un mélange de solvants choisis parmi les solvants aromatiques, les solvants aliphatiques chlorés et les éthers aliphatiques cycliques ou non, à une température comprise entre 10° et 160°C sous une pression égale ou inférieure à 20 bars,pour obtenir un acylsemicarbazide de formule

$$R^1 - \underset{\substack{\| \\ O}}{C} - NH - NR^3 - \underset{\substack{\| \\ O}}{C} - NHR^2 \qquad (V)$$

c) cyclisation du composé de formule (V) au moyen d'une solution aqueuse de soude ou de potasse, éventuellement en présence du ou des solvants de l'étape b), à une température comprise entre 80° et 140°C pour obtenir le composé (I).

Les monoacylhydrazines de formule $R^1 - \underset{\substack{\| \\ O}}{C} - NH - NHR^3$

utilisées comme composés de départ dans l'étape a) sont des composés disponibles dans le commerce ou sont facilement obtenus par différents procédés connus décrits par exemple dans "Methoden der Organischen Chemie" (Houben Weyl) Georg Thienne Verlag 1985 Vol E 5, p. 1154-1170.

Le procédé le plus utilisé consiste à faire réagir un ester de formule $R^1 - \underset{\substack{\| \\ O}}{C} - O\ R$

avec l'hydrate d'hydrazine $NH_2 - NHR^3$, $H_2O$, $R^1 - \underset{\substack{\| \\ O}}{C} - OR + NH_2 - NHR^3$

$, H_2O \rightarrow R^1 - \underset{\substack{\| \\ O}}{C} - NH - NHR^3 + ROH + H_2O$

Les monoacylhydrazines préférées pour la mise en oeuvre du procédé selon l'invention sont celles pour lesquelles $R^1$ représente un atome d'hydrogène, un radical alkyle en $C_1$ à $C_8$, linéaire ou ramifié, substitué ou non par un ou plusieurs substituants choisis parmi les atomes de fluor ou de chlore et le radical phényle ; un radical cycloalkyle, substitué ou non par un ou plusieurs substituants choisis parmi les atomes de fluor ou de chlore, les radicaux alkyles en $C_1$ à $C_4$ et le radical phényle, et $R^3$ représente un atome d'hydrogène, un radical alkyle en $C_1$ à $C_4$, linéaire ou ramifié, un radical phényle substitué ou non par un à deux atomes de fluor, chlore ou brome, et/ou un à deux radicaux alkyle en $C_1$ à $C_4$, et/ou un radical alkoxy linéaire ou ramifié en $C_1$ à $C_4$, et/ou le radical $-CF_3$, et/ou un radical $-COOCH_3$, et/ou un radical de formule $R^4SO_2NH-$ dans laquelle $R^4$ représente un radical méthyle ou éthyle, et/ou un radical $N(CH_3)_2-CO-NH-$, et en particulier celles pour lesquelles $R^1$ représente l'atome d'hydrogène, un radical alkyle en $C_1$ à $C_4$ ou le radical cyclopropyle,et $R^3$ représente un atome d'hydrogène, un radical méthyle, éthyle ou ter-butyle, le radical phényle substitué ou non par un à deux atomes de chlore, et/ou un à deux radicaux méthyle, et/ou un groupe $-NO_2$, et/ou un radical alkoxy en $C_1$ à $C_3$, linéaire ou ramifié.

Les monoacylhydrazines sont mises à réagir avec le phosgène $COCl_2$ selon des procédés décrits par exemple par A. Hetzheim et L. Mockel dans Adv. Heterocyclic Chem (1966) 7, p. 183 ou par G.M. Rosen et Al dans J. Heterocyclic. Chem. (1971) 8 (4) p. 659-662.

On peut également remplacer le phosgène par un de ses dérivés, tel que le chloroformiate d'éthyle ou de benzyle.

Généralement le phosgène est utilisé en excès, en particulier en quantité comprise entre 1,05 et 1,25 équivalent.

Les solvants préférés dans le cadre de l'invention sont choisis parmi les solvants aromatiques tels que le toluène, le xylène, les chlorobenzènes, les solvants aliphatiques chlorés, tels que le dichlorométhane, le 1,2-dichloroéthane, les esters tels que l'acétate d'éthyle.

On effectue la réaction à une température choisie entre 0° et 130°C, de préférence entre 20°C et 80°C. On peut ajouter un accepteur d'acide chlorhydrique tel que la pyridine ou la triéthylamine, la température est alors de préférence comprise entre 0° et 20°C.

La durée de la réaction est généralement comprise entre 2 et 10 heures. L'isolation de l'oxadiazolinone peut

être effectuée mais n'est pas nécessaire.

Les 1,3,4-oxadiazolin-2-ones obtenues à l'étape a) sont ensuite mises à réagir avec une quantité stoechiométrique ou un léger excès d'un composé de formule $R^2NH_2$. Les composés que l'on peut utiliser se trouvent dans le commerce et sont très variées. De préférence $R^2$ représente un atome d'hydrogène, un radical alkyle en $C_1$ à $C_8$, linéaire ou ramifié, substitué ou non par un ou plusieurs substituants choisis parmi les radicaux phényle et phénoxy ; un radical cycloalkyle en $C_3$ à $C_7$, substitué ou non par un ou plusieurs substituants choisis parmi les radicaux alkyle en $C_1$ à $C_4$, phényle et phénoxy ; un radical phényle substitué ou non par un groupe nitro ou phénoxy, et/ou par un à trois substituants choisis parmi les atomes de fluor, chlore ou brome, les radicaux alkyle en $C_1$ à $C_4$ et alkoxy en $C_1$ à $C_4$ ; un radical hétérocyclique à 5 ou 6 chaînons saturé ou insaturé, les hétéroatomes étant choisis parmi l'oxygène, l'azote et le soufre. En particulier $R^2$ représente un atome d'hydrogène, un radical alkyle en $C_1$ à $C_4$, le radical phénoxy-2-éthyle ou le radical phényle substitué ou non par un ou deux atomes de chlore.

Lorsqu'un solvant ou un mélange de solvants sont utilisés, ils sont choisis parmi les solvants aromatiques tel que le toluène, le xylène ou les chlorobenzènes, les solvants aliphatiques chlorés tel que le 1,2-dichloroéthane ou le dichlorométhane ou les éthers aliphatiques cycliques comme le dioxanne.

La température est de préférence comprise entre 40° et 150°C.

La réaction est généralement effectuée à la pression atmosphérique. Dans le cas où le composé de formule $R^2NH_2$ est un gaz, comme par exemple l'ammoniac on préfère opérer sous une pression qui peut aller jusqu'à 20 bars.

La durée de la réaction est généralement comprise entre 2 et 10 heures et le rendement est généralement supérieur à 85 %.

Pour réaliser la cyclisation des semicarbazides obtenus dans l'étape b) on les introduit, éventuellement en présence du ou des solvants de l'étape b), dans une solution aqueuse de soude ou de potasse dont la concentration est comprise entre 1 à 40 % et l'on chauffe le milieu réactionnel à une température de préférence comprise entre 100 et 120°C.

La quantité de soude ou de potasse utilisée est de préférence comprise entre 1,05 et 1,30 équivalent par équivalent d'acylsemicarbazide à cycliser.

La durée de la réaction est généralement comprise entre 1 et 5 heures. On obtient des 1,2,4-triazol-3-ones très pures avec un rendement supérieur à 70 %.

Le procédé selon l'invention permet ainsi, en trois étapes dont la mise en oeuvre est simple, d'obtenir des 1,2,4-triazol-3-ones de grande pureté qui pourront être utilisées directement dans d'autres procédés, sans qu'il soit nécessaire d'effectuer une purification ultérieure. Les produits de départ sont disponibles dans le commerce et sont bon marché. Le rendement global est élevé, supérieur à 60 % par rapport à l'hydrazide de départ.

Les applications des 1,2,4-triazol-3-ones sont connues depuis de nombreuses années. Ce sont des intermédiaires très utiles pour la fabrication de pesticides et herbicides comme décrit dans la demande de brevet WO 86 02642 et la demande de brevet FR 2 535 168, de composés pharmaceutiques comme décrit dans les brevets DE 1 147 591, DE 1 126 882, US 3 857 845, US 4 386 091 et FR 2 555 582 et de polymères comme décrit dans le brevet SU 1 002 290.

Les exemples qui suivent illustrent l'invention.

Exemple 1

Préparation de la 5-éthyl-1,3,4-oxadiazolin-2-one

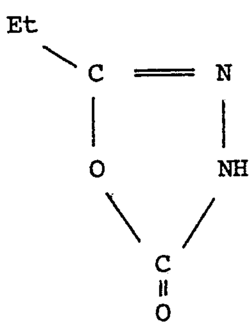

On ajoute lentement, en une heure, 175,8 g (2 moles) de propionylhydrazine Et-$\underset{\underset{O}{\|}}{C}$-NH - $NH_2$ fondu (aux environs de 45°C) à une solution de 100 g de phosgène dans 350 g de toluène, maintenue à 15°C.

On ajoute à nouveau 132,6 g de phosgène (soit 2,35 moles au total), on chauffe le milieu réactionnel à 60° C en une heure et on maintient à cette température pendant 2 heures.

Après élimination de l'excès de phosgène par dégazage à l'azote, on évapore le solvant et on distille le résidu sous pression réduite (Eb. 94-96° C/0,4 mm Hg). On obtient 203 g (rendement 89 %) du produit attendu de pureté 97 % (mesurée par chromatographie en phase gazeuse).

Exemple 2

Préparation de la 4,5-diéthyl-1,2,4-triazol-3-one

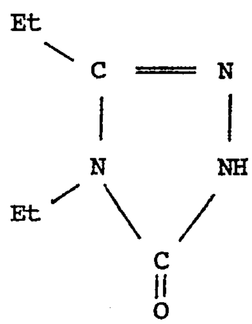

On chauffe à 50-70°C pendant 4 heures une solution de 26,2 g (0,23 mole) de 5-éthyl-1,3,4-oxadiazolin-2-one obtenue à l'exemple 1 et de 17,1 g (0,38 mole) d'éthylamine dans 190 ml de toluène. On précipite le 4-éthyl-1-propionyl-semicarbazide Et-C-NH-NH-C-NH-Et (C=O, C=O) par addition de 200 ml d'hexane, on recueille le précipité par filtration et on sèche sous vide. On obtient 35,9 g (rendement 97 %) d'acylsemicarbazide brut.

On traite 21,2 g (0,133 mole) de cet acylsemicarbazide par une solution de 7,6 g (0,19 mole) de soude dans 30 ml d'eau, à 100°C pendant 1 heure.

On refroidit le mélange à 25° C, on acidifie à pH = 5 par addition d'acide chlorhydrique à 37 % et on extrait avec 3 x 75 ml de chloroforme.

Après élimination du solvant organique par évaporation sous pression réduite, on recristallise le résidu dans 70 ml d'acétate d'éthyle et on sèche à 60°C sous 3 mm Hg. On obtient ainsi 13,7 g (rendement 73 %) du produit attendu, point de fusion : F = 125-127, 5°C.

Exemple 3

Préparation du 4-(2-phénoxyéthyl)-1-propionyl-semicarbazide

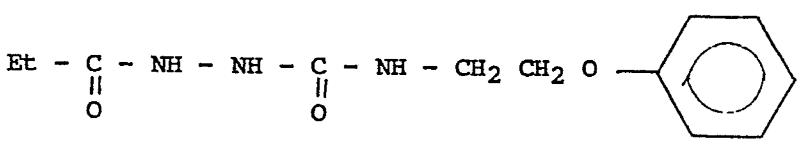

On ajoute 24,3 g (0,177 mole) de 2-phénoxyéthylamine

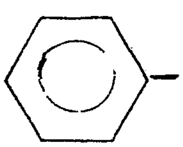 — O - $CH_2CH_2NH_2$ à une solution de 20,05 g (0,176 mole)

à une solution de 20,05 g (0,176 mole) de 5-éthyl-1,3,4-oxadiazolin-2-one obtenue dans l'exemple 1 dans 90 g

de toluène maintenue à 15°C. Après 1 heure d'agitation, on chauffe le milieu réactionnel à 75°C pendant 3 heures.

Après refroidissement à 20°C, on recueille le produit solide par filtration et on le sèche sous pression réduite. On recueille ainsi 39,1 g (rendement 88 %) du produit attendu. Point de fusion : F = 185-187°C (Litt. 180-183°C).

Exemple 4

Préparation de la 4-(2-phénoxyéthyl)-5-éthyl-1,2,4-triazol-3-one

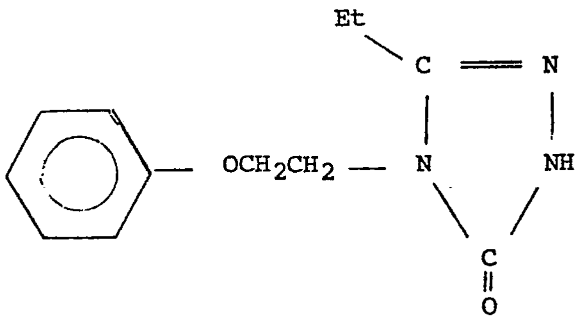

On chauffe à 110°C pendant 1 heure une solution de 10 g (0,0398 mole) de l'acylsemicarbazide de l'exemple 3 et de 2,72 g (0,0485 mole) de potasse dans 50 g d'eau.

On refroidit le milieu à 20°C, on élimine par filtration la faible quantité de solide (0,20 g) et on ajoute au filtrat 7,0 g d'acide chlorhydrique à 37 %. Le précipité formé est recueilli par filtration, lavé avec 2 x 20 ml d'eau distillée et séché sous pression réduite à 60°C sous 1 mm Hg pendant 3 heures. On recueille ainsi 8,37 g (rendement 90 %) du produit attendu sous forme d'un solide blanc fondant à 136,5-137°C (Litt. 137,5 - 138°C). La pureté mesurée par chromatographie en phase gazeuse est de 99,3 %.

Exemple 5

Préparation de la 4-(3,4-dichlorophényl)-5-éthyl-1,2,4-triazol-3-one

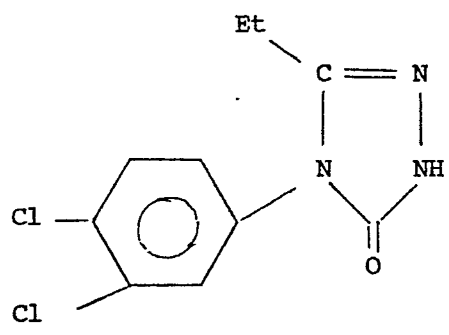

On chauffe à 110° C pendant 8 heures une solution de 5,3 g de 5-éthyl-1,3,4-oxadiazolin-2-one (obtenue dans l'exemple 1) et de dichloro-3,4 aniline (5,5 g) dans 21 g de toluène. On ajoute une solution de 2,9 g de KOH à 85 % dans 36 g d'eau et on agite le mélange à reflux pendant 90 min. Le mélange est refroidi à la température ambiante et le phase toluène est éliminée. On acidifie le mélange aqueux à pH 2 avec de l'acide chlorhydrique à 37 % et on ajoute 200 ml de $CH_2Cl_2$. Le mélange est agité et filtré. La phase $CH_2Cl_2$ est séparée, séchée ($Na_2SO_4$), et concentrée à 80°C sous vide. On obtient 3,4 g (rendement 39 %) du produit attendu avec les caractéristiques spectrales suivantes :

IR ($CH_2Cl_2$, $cm^{-1}$) : 1 705 ( $\nu$ C=O)

RMN $H^1$ (200MHz, $CDCl_3$): 10,7 (s, br, 1H) ; 7,50 (d, J = 8,5, 1H) ; 7,38 (d, J = 2, 1H) ; 7,12 (d de d, J = 8,5, 2, 1H) ; 2,39 (q, J = 7,5, 2H) ; 1,09 (t, J = 7,5, 3H)

RMN $C^{13}$ (200MHz, $CDCl_3$): 155,5 (C=O) ; 148,9 (C=N) ; 140-120 (aromatiques) ; 20,2 ($CH_2$) ; 10,2 ($CH_3$)

Exemple 6

Préparation de la 3-(4-chlorophényl)-1,3,4-oxadiazolin-2-one

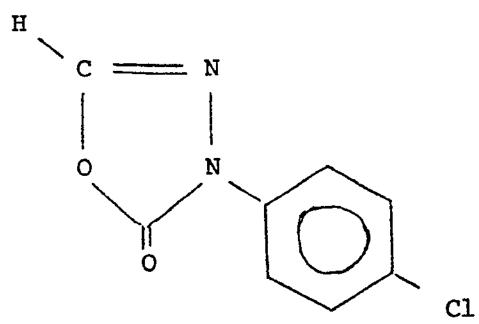

On ajoute du phosgène (25,2 g) à une solution de 1-formyl-2-(4-chlorophényl)hydrazine (35,1 g) dans du chlorobenzène (130 g) à 90-95° C. On chauffe le milieu réactionnel pendant 2 heures à 95-100° C et on élimine l'excès de phosgène et le solvant. On effectue une distillation éclair du produit (Eb. = 140° C/1 mmHg). On obtient 12,7 g (rendement 31 %) du produit attendu ; F = 99-101° C (recristallisation heptane).

IR ($CH_2Cl_2$, $cm^{-1}$) : 1790 ( ν C=O)

RMN $H^1$ ($CDCl_3$) : 8,0-7,5 (m)

Exemple 7

Préparation de la 2-(4-chlorophényl)-1,2,4-triazol-3-one

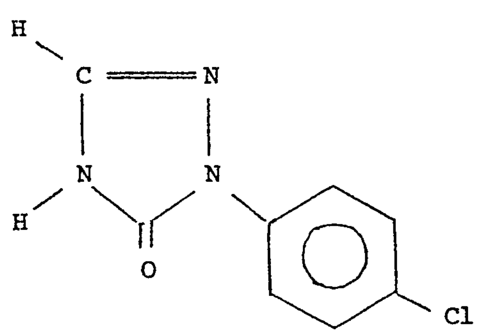

On ajoute de l'ammoniac (6,0 g) à une solution de 3-(4-chlorophényl)-1,3,4-oxadiazolin-2-one (8,3 g) (préparé dans l'exemple 6) dans du dioxane (60 g). Le réacteur est scellé et chauffé à 140° C (la pression augmente jusqu'à 13 atmosphères) pendant 4 heures. Le mélange est refroidi à la temperature ambiante et concentré. On ajoute KOH (3,2 g à 85 %) and $H_2O$ (57 g) et le mélange est chauffé à reflux pendant 90 min, refroidi à température ambiante, et acidifié à pH 2 avec HCl à 37 %. On filtre le produit, on le lave avec de l'eau (3 x 50 ml) et on le sèche sous vide ; on obtient 3,8 g du produit attendu (rendement 46 %) ; F = 260-263° C (recristallisation EtOAc).

IR (KBr, $cm^{-1}$) : 1690 ( $\nu$ C=O)
RMN $H^1$ (DMSO -$d^6$): 12,1 (s, br, 1H) ; 8,19 (s, 1H), 8,0 (d) et 7,5 (d) : système AA'BB'
RMN $C^{13}$ (DMSO-$d^6$): 156,0 (C=O) ; 140,6 (C=N) ; 140,6, 132,8, 132,6 et 123,1 (aromatiques).

**Revendications**

1. Procédé de préparation des 1,2,4-triazol-3-ones de formule générale :

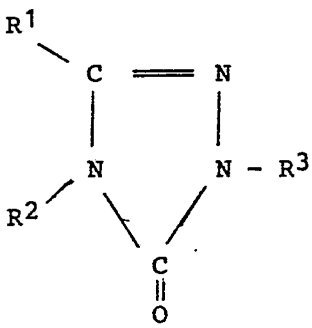

(I)

dans laquelle
$R^1$ représente
- un atome d'hydrogène
- un radical aliphatique en $C_1$ à $C_{20}$, linéaire ou ramifié, substitué ou non par un ou plusieurs substituants choisis parmi les atomes de fluor ou de chlore et le radical phényle,
- un radical cycloaliphatique en $C_3$ à $C_7$, substitué ou non par un ou plusieurs substituants choisis parmi les atomes de fluor ou de chlore, les radicaux aliphatiques en $C_1$ à $C_4$ et phényle,

$R^2$ représente
- un atome d'hydrogène,
- un radical aliphatique en $C_1$ à $C_{20}$, linéaire ou ramifié,substitué ou non par un ou plusieurs substituants choisis parmi les radicaux alkoxy en $C_1$ à $C_4$, phényle et phénoxy,
- un radical cycloaliphatique en $C_3$ à $C_7$, substitué ou non par un ou plusieurs substituants choisis parmi les radicaux aliphatiques en $C_1$ à $C_4$ et alkoxy en $C_1$ à $C_4$, phényle et phénoxy,
- un radical phényle ou naphtyle, substitué ou non par un groupe nitro ou phénoxy et/ou par un à trois substituants choisis parmi les atomes de fluor, chlore ou brome, les radicaux aliphatiques en $C_1$ à $C_4$ et alkoxy en $C_1$ à $C_4$,
- un radical hétérocyclique à 5 ou 6 chaînons, saturé ou insaturé, les hétéroatomes étant choisis parmi l'oxygène, l'azote ou le soufre,

$R^3$ représente
- un atome d'hydrogène,
- un radical alkyle en $C_1$ à $C_6$, linéaire ou ramifié,
- un radical phényle ou naphtyle, substitué ou non par un à trois atomes de fluor, chlore ou brome, et/ou un à trois radicaux alkyle en $C_1$ à $C_4$, et/ou un à deux radicaux alkoxy en $C_1$ à $C_4$, linéaire ou ramifié, et/ou un radical $-CF_3$, et/ou un radical $-COOCH_3$ ou $-COOC_2H_5$, et/ou un à deux groupes $-NO_2$, et/ou un groupe de formule $R^4SO_2NH-$, dans laquelle $R^4$ représente un radical alkyle en $C_1$ à $C_4$, et/ou un groupe de formule $NR^5R^6CONH-$ dans laquelle $R^5$ et $R^6$ représentent des radicaux méthyle ou éthyle,

caractérisé en ce qu'il comprend les étapes consécutives suivantes :

a) réaction d'une monoacylhydrazine de formule $R^1 - \underset{\underset{O}{\|}}{C} - NH - NHR^3$ (II)

dans laquelle $R^1$ et $R^3$ ont les significations précédentes, avec du phosgène, dans un solvant ou un mélange de solvants inertes vis à vis du phosgène et à une température comprise entre 0° et 130° C, pour obtenir une 1,3,4-oxadiazolin-2-one de formule

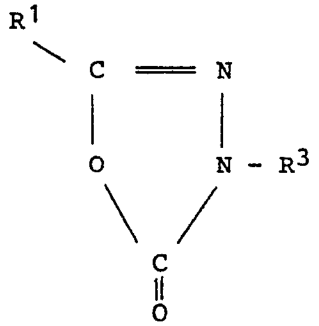

(III)

b) réaction du composé (III) précédemment obtenu avec un composé de formule $R^2NH_2$ (IV) dans laquelle $R^2$ a les significations précédentes, éventuellement dans un solvant ou un mélange de solvants choisis parmi les solvants aromatiques, les solvants aliphatiques chlorés et les éthers aliphatiques cycliques ou non, à une température comprise entre 10° et 160°C et sous une pression inférieure ou égale à 20 bars, pour obtenir un acylsemicarbazide de formule

$R^1-\underset{\underset{O}{\|}}{C}-NH-NR^3-\underset{\underset{O}{\|}}{C}-NHR^2$ (V)

c) cyclisation du composé de formule (V) au moyen d'une solution aqueuse de soude ou de potasse, éventuellement en présence du ou des solvants de l'étape b), à une température comprise entre 80° et 140°C pour obtenir le composé (I).

2) Procédé de préparation selon la revendication 1 caractérisé en ce que $R^1$ représente :
- un atome d'hydrogène,
- un radical alkyle en $C_1$ à $C_8$, linéaire ou ramifié, substitué ou non par un ou plusieurs substituants choisis parmi les atomes de fluor ou de chlore et le radical phényle ;
- un radical cycloalkyle, substitué ou non par un ou plusieurs substituants choisis parmi les atomes de fluor ou de chlore, les radicaux alkyle en $C_1$ à $C_4$ et radical phényle,

et $R^2$ représente :
- un atome d'hydrogène,
- un radical alkyle en $C_1$ à $C_8$, linéaire ou ramifié, substitué ou non par un ou plusieurs substituants choisis parmi les radicaux phényle et phénoxy ;
- un radical cycloalkyle en $C_3$ à $C_7$, substitué ou non par un ou plusieurs substituants choisis parmi les radicaux alkyle en $C_1$ à $C_4$, phényle et phénoxy
- un radical phényle substitué ou non par un groupe nitro ou phénoxy, et/ou par un à trois substituants choisis parmi les atomes de fluor, chlore ou brome, les radicaux alkyle en $C_1$ à $C_4$ et alkoxy en $C_1$ à $C_4$ ;
- un radical hétérocyclique à 5 ou 6 chaînons, saturé ou insaturé, les hétéroatomes étant choisis parmi l'oxygène, l'azote et le soufre

et $R^3$ représente:
- un atome d'hydrogène,
- un radical alkyle en $C_1$ à $C_4$, linéaire ou ramifié,
- un radical phényle substitué ou non par un ou deux atomes de fluor, chlore ou brome, et/ou un à deux radicaux alkyle en $C_1$ à $C_4$, et/ou un radical alkoxy linéaire ou ramifié en $C_1$ à $C_4$, et/ou le radical $-CF_3$, et/ou un radical $-COOCH_3$, et/ou un radical de formule $R^4SO_2NH-$, dans laquelle $R^4$ représente un radical méthyle ou éthyle, et/ou un radical $N(CH_3)_2-CO-NH-$,

3) Procédé de préparation selon la revendication 1 ou 2 caractérisé en ce que $R^1$ représente l'atome d'hydrogène, un radical alkyle en $C_1$ à $C_4$ ou le radical cyclopropyle, $R^2$ représente un atome d'hydrogène, un radical alkyle en $C_1$ à $C_4$, le radical phénoxy-2-éthyle ou le radical phényle substitué ou non par un à deux atomes de chlore et $R^3$ représente un atome d'hydrogène, un radical méthyle, éthyle ou ter-butyle, le radical phényle substitué ou non par un à deux atomes de chlore, et/ou un à deux radicaux méthyle, et/ou un groupe $-NO_2$, et/ou un radical alkoxy en $C_1$ à $C_3$, linéaire ou ramifié.

4) Procédé de préparation selon la revendication 1, 2 ou 3 caractérisé en ce que la réaction a) est effectuée à une température de 20° à 80°C ou de 0° à 20°C lorsque un accepteur d'acide chlorhydrique tel que la pyridine ou la triéthylamine est utilisé, la réaction b) est effectuée à une température comprise entre 40°C et 150°C et la réaction c) à une température comprise entre 100° et 120°C.

5) Procédé selon l'une quelconque des revendications précédentes caractérisé en ce que le solvant ou le mélange de solvants utilisés pour l'étape a) sont choisis parmi les solvants aromatiques tel que le toluène, le xylène et les chlorobenzènes et les solvants aliphatiques chlorés tel que le dichlorométhane et le 1,2-dichloroéthane, les esters tel que l'acétate d'éthyle et le solvant ou le mélange de solvants utilisés dans l'étape b) sont choisis parmi les solvants aromatiques tel que le toluène, le xylène, les chlorobenzènes, les solvants aliphatiques chlorés tel que le 1,2-dichloroéthane ou dichlorométhane, ou les éthers aliphatiques cycliques comme le dioxanne.

6) Procédé selon l'une quelconque des revendications précédentes caractérisé en ce que la quantité de soude ou de potasse utilisée dans l'étape c) est comprise entre 1,05 et 1,30 équivalent par équivalent de semicarbazide à cycliser.

7) Procédé selon l'une quelconque des revendications précédentes caractérisé en ce que dans l'étape a) on remplace le phosgène par du chloroformiate d'éthyle ou de benzyle.

Office européen des brevets

# RAPPORT DE RECHERCHE EUROPEENNE

Numero de la demande

EP 88 40 1892

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | JOURNAL OF HETEROCYCLIC CHEMISTRY, vol. 19, juillet/août 1982, pages 823-828, Provoh, Utah, US; K.H. PILGRAM: "5-Substituted-3-(substituted)phenyl-1,3,4-oxadiazolin-2(3H)-ones. Synthesis and reactions with nucleophilic reagents" * En entier, en particulier page 823, colonne de gauche, dernier paragraphe; page 824, colonne de gauche, paragraphe 1; page 826, colonne de gauche, paragraphe 1 * --- | 1-7 | C 07 D 249/12 |
| Y | FR-A-1 470 310 (HOECHST) * En entier, en particulier résumé, point 2h * --- | 1-7 | |
| D,Y | J. LIEBIGS: "Annalen der Chemie", vol. 675, 24 juillet 1964, pages 180-188, Verlag Chemie GmbH, Weinheim, DE; H. GEHLEN et al.: "Bildung von 1.2.4-Triazolonen-(5) aus substituierten Acylsemicarbaziden" * En entier * ----- | 1-7 | |
| | | | **DOMAINES TECHNIQUES RECHERCHES (Int. Cl.4)** |
| | | | C 07 D 249/00<br>C 07 D 271/00 |

Le présent rapport a été établi pour toutes les revendications

| Lieu de la recherche | Date d'achèvement de la recherche | Examinateur |
|---|---|---|
| LA HAYE | 20-10-1988 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)